# EUROPÄISCHE PATENTANMELDUNG

(11) **EP 4 098 646 A1**
(43) Veröffentlichungstag der Anmeldung: **07.12.2022**
(21) Anmeldenummer: 21177380.9
(22) Anmeldetag: 02.06.2021
(51) Int. Cl.: C07C 47/02, C07F 15/06

(54) **VERFAHREN ZUR HYDROFORMYLIERUNG VON OLEFINEN MIT EINEM COBALT-PRÄKATALYSATOR**

(71) Anmelder: Evonik Operations GmbH, 45128 Essen (DE)
(72) Erfinder: ZHANG, Baoxin, 18106 Rostock (DE); KUBIS, Christoph, 18211 Nienhagen (DE); BÖRNER, Armin, 18059 Rostock (DE); FRANKE, Robert, 45772 Marl (DE)
(74) Vertreter: Evonik Patent Association

(57) **Zusammenfassung**

Verfahren zur Hydroformylierung von Olefinen mit einem Cobalt-Präkatalysator.

## Beschreibung

Die Erfindung betrifft ein Verfahren zur Hydroformylierung von Olefinen mit einem Cobalt-Präkatalysator.

Die technische Aufgabe, welche der vorliegenden Erfindung zugrunde lag, ist die Bereitstellung eines Verfahrens, mit welchen Olefine hydroformyliert werden können. In dem Verfahren soll eine gesteigerte Ausbeute erzielt werden.

Diese Aufgabe wird gelöst durch ein Verfahren nach Anspruch 1.

Verfahren umfassend die Verfahrensschritte:
a) Zugabe eines Olefins;
b) Zugabe eines Cobalt-Präkatalysators,
   wobei es sich bei dem Cobalt-Präkatalysators um [Co(acac)(C₄H₈O₂)₄]⁺[BF₄]⁻ handelt;
c) Zuführen von Synthesegas;
d) Erwärmen des Reaktionsgemisches aus a) bis c), wobei das Olefin zu einem Aldehyd umgesetzt wird.

Hierbei können die Verfahrensschritte a), b) und c) in beliebiger Reihenfolge erfolgen.

In einer Variante des Verfahrens ist das Olefin ausgewählt aus:
Ethen, Propen, 1-Buten, cis-2-Buten, trans-2-Buten, Gemisch aus cis- und trans-2-Buten, Raffinat 1, Raffinat 2, Isobuten, 1-Penten, 2-Penten, 2-Methyl-1-buten, 2-Methyl-2-buten, 1-Methylcyclohexen, Tetramethylethen, 1-Octen, 2-Octene, Cycloocten, Di-n-Buten, Di-iso-Buten, Undecene, Dodecene, Triisobuten, Tri-n-Buten.

In einer Variante des Verfahrens ist das Olefin ausgewählt aus:
1-Buten, cis-2-Buten, trans-2-Buten, Isobuten, 1-Penten, 2-Penten, 1-Octen, 2-Octene, Di-n-Buten, Di-iso-Buten, Triisobuten, Tri-n-Buten.

In einer Variante des Verfahrens wird der Cobalt-Präkatalysator als Lösung vorgelegt.

In einer Variante des Verfahrens ist wird das Olefin zu der vorgelegten Cobalt-Präkatalysator-Lösung zugegeben.

In einer Variante des Verfahrens erfolgt das Zuführen von Synthesegas in Verfahrensschritt c) mit einem Druck in dem Bereich von 1 bis 8 MPa (10 bis 80 bar).

In einer Variante des Verfahrens erfolgt das Zuführen von Synthesegas in Verfahrensschritt c) mit einem Druck in dem Bereich von 4 bis 6 MPa (40 bis 60 bar).

In einer Variante des Verfahrens erfolgt das Erwärmen des Reaktionsgemisches in Verfahrensschritt d) auf eine Temperatur in dem Bereich von 80 °C bis 180 °C.

In einer Variante des Verfahrens erfolgt das Erwärmen des Reaktionsgemisches in Verfahrensschritt d) auf eine Temperatur in dem Bereich von 120 °C bis 160 °C.

Im Folgenden soll die Erfindung anhand eines Ausführungsbeispiels näher erläutert werden.

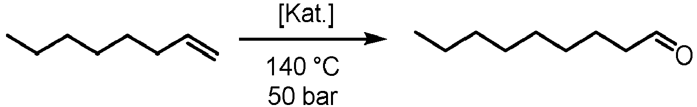

Eine Lösung des Präkatalysators (40,1 mL) wird in einen 300 mL Premex Autoklaven mit Begasungsrührer versetzt, und ist mit Synthesegas auf 35 bar beschickt. Die Lösung wird bis auf 160 °C aufgeheizt und der Druck auf 50 bar nachreguliert. Nach 30 Minuten wird die Temperatur auf 140 °C abgesenkt. Nun wird das Olefin (1-Octen) durch eine Spritzenpumpe zur Katalysatorlösung zugefügt, und die Reaktionslösung 3 Stunden bei 50 bar bei 140 °C gerührt. Dann wird die Reaktionsmischung auf Raumtemperatur abgekühlt und der Druck abgelassen. Die Ausbeute wird durch Gaschromatographie bestimmt.

Die Rektion wurde mit drei unterschiedlichen Präkatalysatoren durchgeführt.
Bei den Präkatalysatoren (**1**) und (**2**) handelt es sich um Vergleichs-Präkatalysatoren.

### Präkatalysatoren:

(**1**) Co₂(CO)₈

(**2**) Co(acac)₂

(**3**) [Co(acac)(C₄H₈O₂)₄]⁺[BF₄]⁻

### Reaktionsbedingungen:

T = 140 °C, p(Synthesegas) = 50 bar, t = 3 h, Olefin = 1-Octen

Die Versuchsergebnisse sind in der nachfolgenden Tabelle aufgeführt:

| Präkatalysator | Olefin | [Co] (mM) | T (°C) | t (h) | Ausbeute (%) |
|---|---|---|---|---|---|
| (**1**) | 1-Octen | 1 | 140 | 3 | 62,7 |
| (**2**) | 1-Octen | 1 | 140 | 3 | 66,9 |
| (**3**)* | 1-Octen | 1 | 140 | 3 | 69,3 |

| | | | | | |
|---|---|---|---|---|---|
| * erfindungsgemäßes Verfahren | | | | | |

In dem erfindungsgemäßen Verfahren mit dem Präkatalysator (**3**) konnte eine bessere Ausbeute erzielt werden als in den beiden Vergleichsversuchen.

Wie das Ausführungsbeispiel zeigt, wird die Aufgabe durch das erfindungsgemäße Verfahren gelöst.

## Patentansprüche

1. Verfahren umfassend die Verfahrensschritte:
a) Zugabe eines Olefins;
b) Zugabe eines Cobalt-Präkatalysators,
wobei es sich bei dem Cobalt-Präkatalysators um [Co(acac)(C₄H₈O₂)₄]⁺[BF₄]⁻ handelt;
c) Zuführen von Synthesegas;
d) Erwärmen des Reaktionsgemisches aus a) bis c), wobei das Olefin zu einem Aldehyd umgesetzt wird.

2. Verfahren nach Anspruch 1,
wobei das Olefin ausgewählt ist aus:
Ethen, Propen, 1-Buten, cis-2-Buten, trans-2-Buten, Gemisch aus cis- und trans-2-Buten, Raffinat 1, Raffinat 2, Isobuten, 1-Penten, 2-Penten, 2-Methyl-1-buten, 2-Methyl-2-buten, 1-Methylcyclohexen, Tetramethylethen, 1-Octen, 2-Octene, Cycloocten, Di-n-Buten, Di-iso-Buten, Undecene, Dodecene, Triisobuten, Tri-n-Buten.

3. Verfahren nach einem der Ansprüche 1 oder 2,
wobei das Olefin ausgewählt ist aus:
1-Buten, cis-2-Buten, trans-2-Buten, Isobuten, 1-Penten, 2-Penten, 1-Octen, 2-Octene, Di-n-Buten, Di-iso-Buten, Triisobuten, Tri-n-Buten.

4. Verfahren nach einem der Ansprüche 1 bis 3,
wobei der Co-Präkatalysator als Lösung vorgelegt wird.

5. Verfahren nach Anspruch 4,
wobei das Olefin zu der vorgelegten Co-Präkatalysator-Lösung zugegeben wird.

6. Verfahren nach einem der Ansprüche 1 bis 5,
wobei das Zuführen von Synthesegas in Verfahrensschritt c) mit einem Druck in dem Bereich von 1 bis 8 MPa (10 bis 80 bar) erfolgt.

7. Verfahren nach einem der Ansprüche 1 bis 6,
wobei das Zuführen von Synthesegas in Verfahrensschritt c) mit einem Druck in dem Bereich von 4 bis 6 MPa (40 bis 60 bar) erfolgt.

8. Verfahren nach einem der Ansprüche 1 bis 7,
wobei das Erwärmen des Reaktionsgemisches in Verfahrensschritt d) auf eine Temperatur in dem Bereich von 80 °C bis 180 °C erfolgt.

9. Verfahren nach einem der Ansprüche 1 bis 8,
wobei das Erwärmen des Reaktionsgemisches in Verfahrensschritt d) auf eine Temperatur in dem Bereich von 120 °C bis 160 °C erfolgt.

## Geänderte Patentansprüche

### Geänderte Patentansprüche gemäss Regel 137(2) EPÜ.

1. Verfahren umfassend die Verfahrensschritte:
a) Zugabe eines Olefins, welches ausgewählt ist aus: 1-Buten, cis-2-Buten, trans-2-Buten, Isobuten, 1-Penten, 2-Penten, 1-Octen, 2-Octene, Di-n-Buten, Di-iso-Buten, Triisobuten, Tri-n-Buten;
b) Zugabe eines Cobalt-Präkatalysators,
wobei es sich bei dem Cobalt-Präkatalysators um [Co(acac)(C₄H₈O₂)₄]⁺[BF₄]⁻ handelt;
c) Zuführen von Synthesegas;
d) Erwärmen des Reaktionsgemisches aus a) bis c), wobei das Olefin zu einem Aldehyd umgesetzt wird.

2. Verfahren nach Anspruch 1,
wobei der Co-Präkatalysator als Lösung vorgelegt wird.

3. Verfahren nach Anspruch 2,
wobei das Olefin zu der vorgelegten Co-Präkatalysator-Lösung zugegeben wird.

4. Verfahren nach einem der Ansprüche 1 bis 3,
wobei das Zuführen von Synthesegas in Verfahrensschritt c) mit einem Druck in dem Bereich von 1 bis 8 MPa (10 bis 80 bar) erfolgt.

5. Verfahren nach einem der Ansprüche 1 bis 4,
wobei das Zuführen von Synthesegas in Verfahrensschritt c) mit einem Druck in dem Bereich von 4 bis 6 MPa (40 bis 60 bar) erfolgt.

6. Verfahren nach einem der Ansprüche 1 bis 5,
wobei das Erwärmen des Reaktionsgemisches in Verfahrensschritt d) auf eine Temperatur in dem Bereich von 80 °C bis 180 °C erfolgt.

7. Verfahren nach einem der Ansprüche 1 bis 6,
wobei das Erwärmen des Reaktionsgemisches in Verfahrensschritt d) auf eine Temperatur in dem Bereich von 120 °C bis 160 °C erfolgt.
